Europäisches Patentamt

European Patent Office

Office européen des brevets

(11) Veröffentlichungsnummer: **0 229 193**
**B1**

(12) # EUROPÄISCHE PATENTSCHRIFT

(45) Veröffentlichungstag der Patentschrift:
18.10.89

(21) Anmeldenummer: 85114598.7

(22) Anmeldetag: 16.11.85

(51) Int. Cl.⁴: **A61N 1/06**, F21V 21/26,
A61N 5/00

(54) Haltevorrichtung für Hochfrequenz-Applikatoren an Hochfrequenz-Therapiegeräten.

(43) Veröffentlichungstag der Anmeldung:
22.07.87 Patentblatt 87/30

(45) Bekanntmachung des Hinweises auf die Patenterteilung:
18.10.89 Patentblatt 89/42

(84) Benannte Vertragsstaaten:
DE GB NL

(56) Entgegenhaltungen:
EP-A- 0 048 402
DE-A- 2 452 851
DE-A- 2 640 863
GB-A- 982 110

(73) Patentinhaber: Erbe Elektromedizin GmbH.,
Ebertstrasse 35, D-7400 Tübingen(DE)

(72) Erfinder: Farin, Günter, Kapellenweg 15,
D-7400 Tübingen(DE)
Erfinder: Fischer, Klaus, Immengasse 1,
D-7270 Nagold-Emmingen(DE)

(74) Vertreter: Endlich, Fritz, Dipl.-Phys.,
Postfach 1326 Blumenstrasse 8, D-8034 Germering(DE)

**Beschreibung**

Die Erfindung betrifft eine Haltevorrichtung für Hochfrequenz-Applikatoren an Hochfrequenz-Therapiegeräten, insbesondere für die Mikrowellen-, Dezimeterwellen- und Kurzwellen-Therapie.

Bei Hochfrequenz-Therapiegeräten sind inzwischen verschiedene Haltevorrichtungen für unterschiedliche Hochfrequenz-Applikatoren bekannt. Man kann die bekannten Haltevorrichtungen bezüglich ihrer Konstruktion in zwei Gruppen unterteilen, und zwar in Haltevorrichtungen, welch aus mehr oder weniger vielen starren, geraden oder abgewinkelten Elementen bestehen, die mit mehr oder weniger vielen Gelenken und teleskopartigen Elementen ausgestattet sind, im folgenden kurz Gelenkarme genannt, und Haltevorrichtungen, welche aus flexiblen Elementen ohne zusätzliche Gelenke oder teleskopartigen Elementen bestehen, im folgenden kurz biegsame Haltearme genannt. Außerdem sind auch Haltevorrichtungen bekannt, bei welchen starre Elemente, Gelenke und/oder flexible Elemente miteinander kombiniert sind.

In dem DP 111 0337 ist ein typischer Gelenkarm beschrieben. In dem EP 00 34 826 AI ist ein typischer biegsamer Haltearm beschrieben. In dem EP 00 34 826 AI ist auch ein Haltearm beschrieben, bei welchem starre Elemente, Gelenke und flexible Elemente miteinander kombiniert sind.

Gelenkarme haben den Nachteil, daß die Zuleitung der elektrischen Energie vom Hochfrequenz-Therapiegerät zu den Hochfrequenz-Applikatoren außerhalb des Gelenkarmes erfolgen muß, es sei denn, es werden innerhalb aller Gelenke elektrische Drehkupplungen angeordnet, was jedoch insbesondere bei Dezimeter- und Mikrowellen-Therapiegeräten, bei denen die elektrische Energie nur über koaxiale Leitungen geleitet werden kann, sehr aufwendig wäre.

Biegsame Haltearme haben im Vergleich zu Gelenkarmen zwar den Vorteil, daß die Zuleitung der elektrischen Energie innerhalb dieser Arme angeordnet werden kann, aber den Nachteil, daß die bekannten flexiblen Elemente keine größeren Gewichte sicher halten können.

Haltevorrichtungen, welche sowohl mit starren Elementen und Gelenken als auch mit flexiblen Elementen ausgestattet sind, haben auch die oben genannten Probleme dieser Elemente.

Ein gemeinsamer Nachteil aller bekannten Haltevorrichtungen bei Hochfrequenz-Therapiegeräten besteht in dem relativ großen Raumbedarf, sowohl in unbenutztem Zustand als insbesondere auch während der Anwendung. Für die Behandlung von Patienten stehen in den Arztpraxen und Krankenhäusern in der Regel nur sehr kleine Behandlungskabinen zur Verfügung, innerhalb welcher oft nur 2 Quadratmeter freie Fläche vorhanden ist, auf welcher das Hochfrequenz-Therapiegerät angewendet werden muß.

Außerdem haben bekannte Haltevorrichtungen von Hochfrequenz-Therapiegeräten den Nachteil, daß sie infolge ihrer sperrigen Konstruktion für den Versand vom Hochfrequenz-Therapiegerät abmontiert und beim Empfänger wieder anmontiert werden müssen, wofür entsprechende Kenntnisse, Werkzeuge und Montageanleitungen erforderliche sind. In vielen Fällen muß deswegen ein Monteur vom Hersteller oder Fachhändler zum Kunden reisen.

Es ist deshalb Aufgabe der Erfindung, einer Haltevorrichtung der genannten Art unter möglichst weitgehender Vermeidung der genannten Nachteile und Schwierigkeiten derart zu verbessern, daß in einer größeren Vielfalt von Drehlagen bei möglichst geringem Raumbedarf eine ausreichend stabile Halterung des Applikators in der eingestellten Lage und eine betriebssichere Führung der Hochfrequenzleitung erzielbar ist.

Gemäß der Erfindung ist ein starrer Haltearm an einem Lager mit horizontaler Drehachse befestigt, wobei das Lager an einer Seitenwand angeordnet ist und vorzugsweise das Drehmoment entsprechend dem Gewicht des Haltearmes und dem Gewicht der verschiedenen Hochfrequenz-Applikatoren multipliziert mit der horizontalen Entfernung des gemeinsamen Schwerpunktes von der horizontalen Drehachse des Lagers innerhalb des Hochfrequenz-Therapiegerätes kompensiert wird, und zwar so, daß der starre Haltearm sowohl mit als auch ohne Hochfrequenz-Applikatoren in allen vorgesehenen Winkelstellungen ausreichend sicher fixierbar ist. Da der horizontale Abstand zwischen gemeinsamem Schwerpunkt und der horizontalen Drehachse des Lagers beim Schwenken des Haltearmes um die horizontale Achse des Lagers einer Cosinusfunktion folgt, ist das Gegendrehmoment innerhalb des Hochfrequenz-Therapiegerätes so gestaltet, daß es ebenfalls einer Cosinusfunktion folgt, und zwar so, daß es bei vertikaler Stellung des Haltearmes Null wird und beim Schwenken des Haltearmes nach links oder rechts in Richtung der jeweiligen Horizontalen bei gleichzeitiger Berücksichtigung des Vorzeichens entsprechend der Cosinusfunktion zunimmt und beim weiteren Schwenken unter die Horizontale wieder entsprechend der Cosinusfunktion abnimmt.

Das Gegendrehmoment, welches innerhalb des Hochfrequenz-Therapiegerätes angeordnet ist, wird durch eine konstante Kraft, beispielsweise einer Druckluftfeder, erzeugt, welche an einem Hebelarm angreift, der um dieselbe horizontale Drehachse des Lagers schwenkt wie der Haltearm.

Mit Rücksicht auf das unterschiedliche Gewicht der verschiedenen Hochfrequenz-Applikatoren und um zu verhindern, daß der Haltearm zu leicht aus der jeweils fixierten Stellung herausbewegt werden kann, kann die Drehbewegung im Lager mittels einer konstanten Reibungsbremse oder mittels einer Feststellbremse in jeder Stellung stabilisiert werden.

Sowohl das Lager als auch der starre Haltearm sind hohl, so daß die Energieleitung vom Hochfrequenzgenerator des Hochfrequenz-Therapiegerätes zu den Hochfrequenz-Applikatoren hierin angeordnet werden kann.

Die vorliegende Haltevorrichtung für Hochfrequenz-Applikatoren an Hochfrequenz-Therapiegeräten besteht grob unterteilt aus dem außerhalb des Gehäuses des Hochfrequenz-Therapiegerätes angeordneten Haltearm und den innerhalb dieses Gehäuses angeordneten Bestandteilen, welche den Haltearm inklusive der verschiedenen Hochfrequenz- Applikatoren im Gleichgewicht halten und unbeabsichtigtes Verstellen des Haltearmes verhindern sollen.

Ausführungsbeispiele der Erfindung sind in Zeichnungen dargestellt und im folgenden näher beschrieben.

Fig. 1 bis Fig. 3 zeigen schematisch ein Hochfrequenz-Therapiegerät mit den Bestandteilen des erfindungsgemäßen Haltearmes, welche sichtbar außerhalb des Gehäuses des Hochfrequenz-Therapiegerätes angeordnet sind.

Fig. 4 zeigt schematisch die Bestandteile eines Ausführungsbeispieles der erfindungsgemäßen Haltevorrichtung, welche innerhalb des Gehäuses des Hochfrequenz-Therapiegerätes angeordnet sind.

Fig. 5 zeigt die Funktion der gekröpften Stange, welche bereits in Fig. 4 in einer Stellung dargestellt ist.

Fig. 6 zeigt schematisch die Bestandteile eines weiteren Ausführungsbeispieles der erfindungsgemäßen Haltevorrichtung, welche innerhalb des Gehäuses des Hochfrequenz-Therapiegerätes angeordnet sind.

Fig. 7 zeigt detaillierter die Bestandteile des Drehlagers mit der Reibungsbremse.

Fig. 8 zeigt detailliert das axiale Drehgelenk im Bereich $l_2$ des Haltearmes.

Fig. 9 zeigt die Kupplung für die Hochfrequenz-Applikatoren.

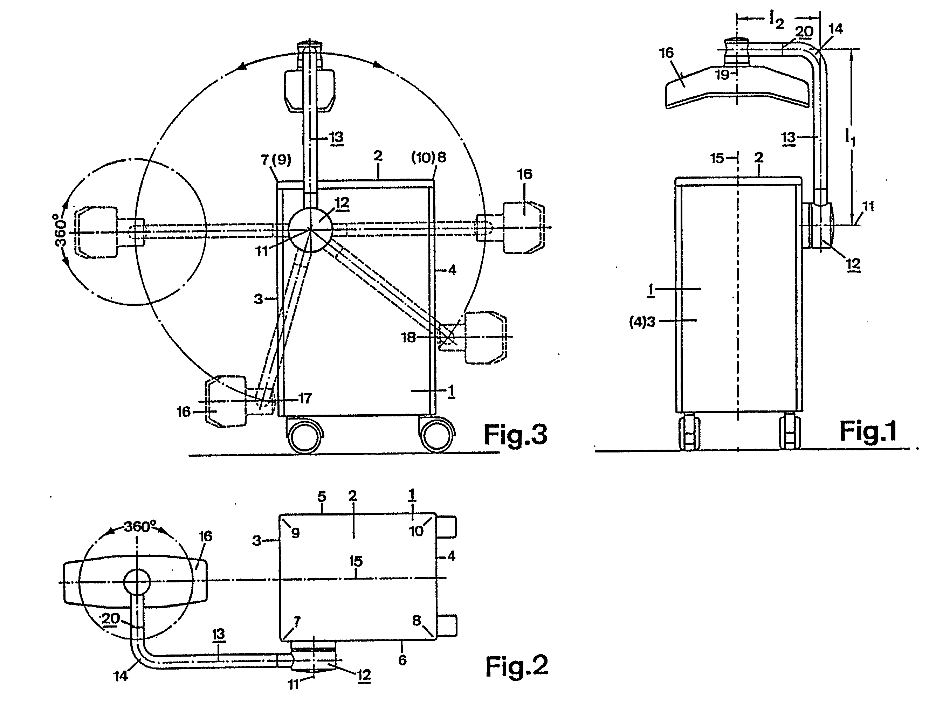

Fig. 1 bis Fig. 3 zeigen eine typische Gehäuseform 1 von fahrbaren Hochfrequenz-Therapiegeräten. Diese Gehäuse sind in der Regel quaderförmig und haben eine rechteckige Bedienungsplatte 2. Bei bekannten Hochfrequenz-Therapiegeräten sind die Haltearme in der Regel an einer der beiden schmalen Seitenwände 3 oder 4 angebracht. Der Haltearm 13 wird dagegen bevorzugt an einer der beiden Breitseiten 5 oder 6 angebracht, und zwar möglichst nahe einer der jeweils beiden Ecken 7 oder 8 bzw. 9 oder 10 der Breitseiten, wobei die Ecken 9 und 10 in Fig. 3 hinter den Ecken 7 und 8 liegen und daher in Klammern gestellt sind. Bei bekannten Hochfrequenz-Therapiegeräten beginnen die Haltearme geräteseitig stets mit einem Gelenk mit vertikaler Drehachse, so daß der gesamte Haltearm bekannter Hochfrequenz-Therapiegeräte gerätenahe um diese vertikale Achse horizontal gedreht werden kann. Der Haltearm 13 beginnt geräteseitig mit einem Drehgelenk 12 mit horizontaler Drehachse 11, so daß der gesamte Haltearm 13 um diese horizontale Drehachse 11 vertikal gedreht werden kann.

Der an dem Drehgelenk 12 angebrachte Haltearm 13 besteht aus einem Rohr, das an der Stelle 14 in Richtung Mittelachse 15 des Gehäuses 1 L-förmig abgewinkelt ist. Die Länge $l_1$ des Haltearmes 13 ist vorzugsweise so bemessen, daß der Hochfrequenz-Applikator 16 bis an die untere Ecke 17, welche in Fig. 3 unterhalb der oberen Ecke 7 liegt, geschwenkt werden kann und zwar so, daß er fast den Fußboden erreicht. In dieser Stellung des Hochfrequenz-Applikators 16 können beispielsweise bei geringstem Platzbedarf die Füße von sitzenden Patienten bestrahlt werden. Wird der Haltearm 13 auf die gegenüberliegende Schmalseite 4 geschwenkt, wo er die Schmalseite 4 an der Stelle 18 berührt, so kann in dieser Stellung des Hochfrequenz-Applikators 16 bei geringstem Platzbedarf der Kniebereich und der Hüftbereich von sitzenden Patienten bestrahlt werden. Zwischen diesen beiden, in Fig. 3 gestrichelt dargestellten, extremen Stellungen 17 und 18 kann der Haltearm 13 und damit der HochfrequenzApplikator 16 in jeder beliebigen Stellung verwendet werden.

Die Länge $l_2$ des Haltearmes 13 ist so bemessen, daß die Mittelachse 19 des Hochfrequenz-Applikators vorzugsweise durch die Mittelachse 15 der Schmalseiten 3, 4 bzw. der Bedienungsplatte 2 läuft.

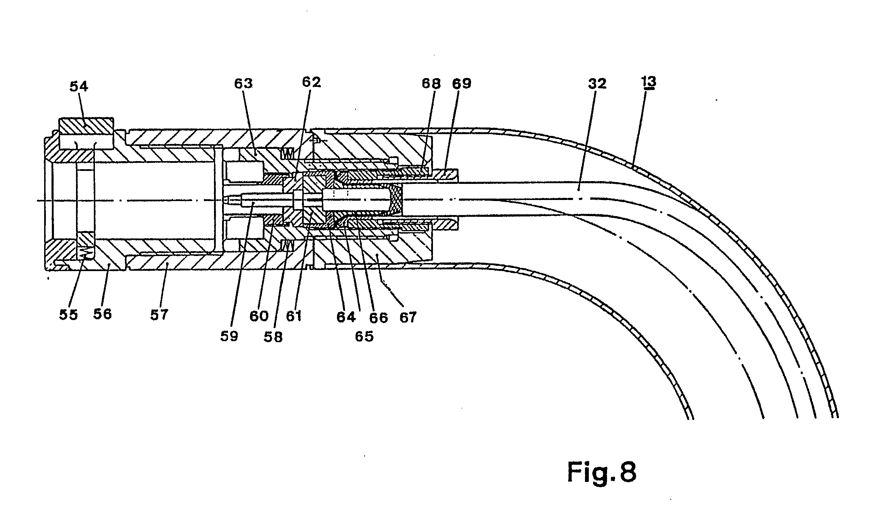

Im Bereich $l_2$ des Haltearmes 13 ist ein Drehgelenk 20 angeordnet, das in Fig. 8 detaillierter dargestellt ist und weiter unten näher beschrieben wird. Um dieses Drehgelenk kann der Hochfrequenz-Applikator 16 um 360° geschwenkt werden, wie in Fig. 3 dargestellt. Wenn auch der Hochfrequenz-Applikator 16, wie in Fig. 2 dargestellt, um mindestens 180° oder gar um 360° geschwenkt werden kann, so ermöglicht dieser erfindungsgemäße Haltearm alle bei sitzenden oder auf einer Behandlungsliege liegenden Patienten vorkommenden Applikationen.

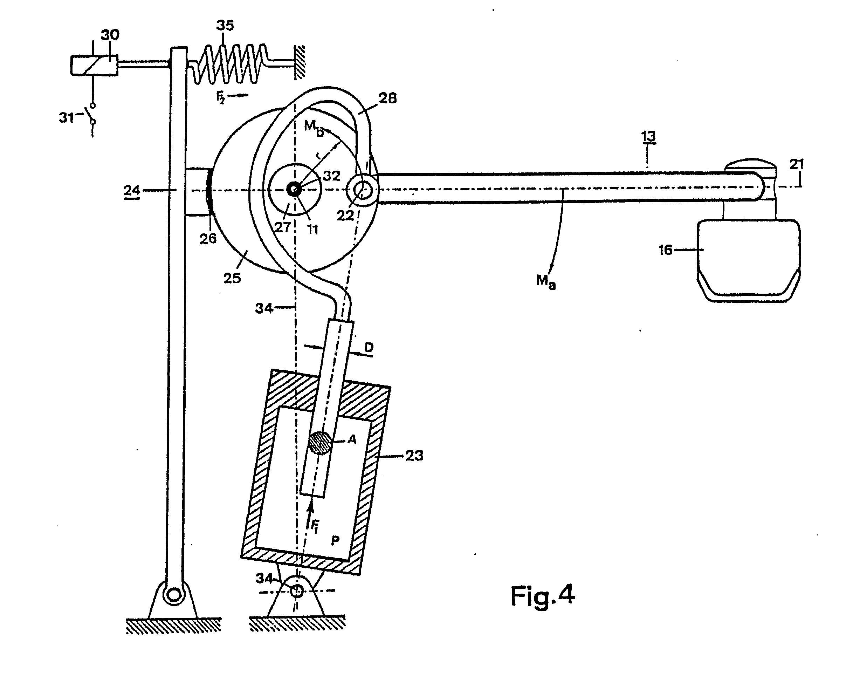

In Fig. 4 sind schematisch die Bestandteile eines Ausführungsbeispieles der erfindungsgemäßen Haltevorrichtung dargestellt, welche innerhalb des Gehäuses des Hochfrequenz-Therapiegerätes 1 angeordnet sind und im folgenden näher beschrieben werden.

Innerhalb des Gehäuses 1 ist eine runde Scheibe 25 angeordnet, welche auf der horizontalen Drehachse 11 drehbar gelagert ist. An dieser Scheibe 25 greift im Drehpunkt 22, welcher im Abstand r von der horizontalen Drehachse 11 angeordnet ist, eine für diesen Zweck ausreichend konstante Kraft $F_1$ an. Der Drehpunkt 22 liegt auf der Achse 21 des Haltearmes 13. Die Kraft $F_1$ wird in einer Druckluftfeder 23 erzeugt. Die Kraft $F_1$ ist proportional dem Querschnitt $A=D^2\pi/4$ und dem Luftdruck P innerhalb der Druckluftfeder 23.

Die Kraft $F_1$ erzeugt auf die Drehachse 11 ein Drehmoment $M_b$, welches proportional ist der Kraft $F_1$ und dem horizontalen Abstand des Drehpunktes 22 von der vertikalen Mittellinie 34 durch den Drehpunkt der Scheibe 25. Wenn das Lager 34 der Druckluftfeder 23 ausreichend weit von der Drehachse 11 entfernt ist, folgt das Drehmoment $M_b$ ausreichend genau einer Cosinusfunktion, wobei dieses Drehmoment $M_b$ jeweils am größten ist, wenn der Haltearm 13 links oder rechts der Drehachse 11 horizontal ausgerichtet ist. Das Drehmoment $M_b$ ist Null, wenn der Haltearm 13 genau vertikal ausgerichtet ist. Das Drehmoment $M_b$ ist stets dem Drehmoment $M_a$ des Haltearmes 13, mit oder ohne Applikator 16, entgegengerichtet.

Bei geeigneter Dimensionierung der Druckluftfeder 23 sowie des Abstandes r des Drehpunktes 22 von der Drehachse 11 heben sich die beiden Drehmomente $M_a$ und $M_b$ weitgehend gegeneinander auf. Um zu verhindern, daß der Haltearm 13 infolge verschiedener Toleranzen, beispielsweise bei Verwendung unterschiedlich schwerer Applikatoren, aber insbesondere auch dann, wenn kein Applikator am Haltearm 13 befestigt ist, aus dem Gleichgewicht gerät und sind automatisch in eine unbeabsichtigte stabile Position bewegt, ist eine Reibungsbremse 24 vorhanden.

Die Reibkraft der Reibungsbremse 24 muß so dimensioniert sein, daß alle vorgesehenen, verschiedenen Applikatoren in allen möglichen Positionen des Haltearmes 13 sicher fixiert werden können. Die Reibkraft resultiert aus der Kraft $F_2$ und dem Reibungskoeffizienten zwischen der Bremsscheibe 25 und der Bremsbacke 26, wobei das Übersetzungsverhältnis des Hebels 24 zu berücksichtigen ist.

In weiterer Ausgestaltung der Erfindung kann die Kraft $F_2$, welche beispielsweise mittels einer Zugfeder 35 erzeugt werden kann, so hoch dimensioniert werden, daß der Haltearm 13 nur mit großer Kraft aus seiner jeweiligen Position herausbewegt werden kann. Hierdurch kann ein unbeabsichtigtes Verstellen des Haltearmes 13 verhindert werden, wenn der Patient versehentlich den Applikator oder den Haltearm berührt. Zur möglichst leichten Einstellung des Haltearmes 13 in eine neue Position ist ein elektrischer Hubmagnet vorhanden, welcher die Kraft $F_2$ entweder verringert oder ganz aufhebt, wenn die Taste 31, welche beispielsweise auf der Bedienungsplatte 2 oder am Haltearm 13 angeordnet sein kann, betätigt wird.

In einfachster Ausführung, beispielsweise für preiswertere Geräte, kann auf die beschriebenen Elemente für Ausgleich des Drehmomentes $M_a$ durch das Gegendrehmoment $M_b$ verzichtet werden, wenn beispielsweise die Taste 31 der Reibungsbremse am Haltearm angeordnet ist, so daß der Haltearm 13 nur dann leicht um die Drehachse 11 gedreht werden kann, wenn die Taste 31 betätigt wird. Hierbei kann der Haltearm 13 sich nicht selbstständig in eine beliebige stabilere Position hineinbewegen, da immer dann, wenn die Taste 31 wieder geöffnet ist, die Bremse 24 anzieht.

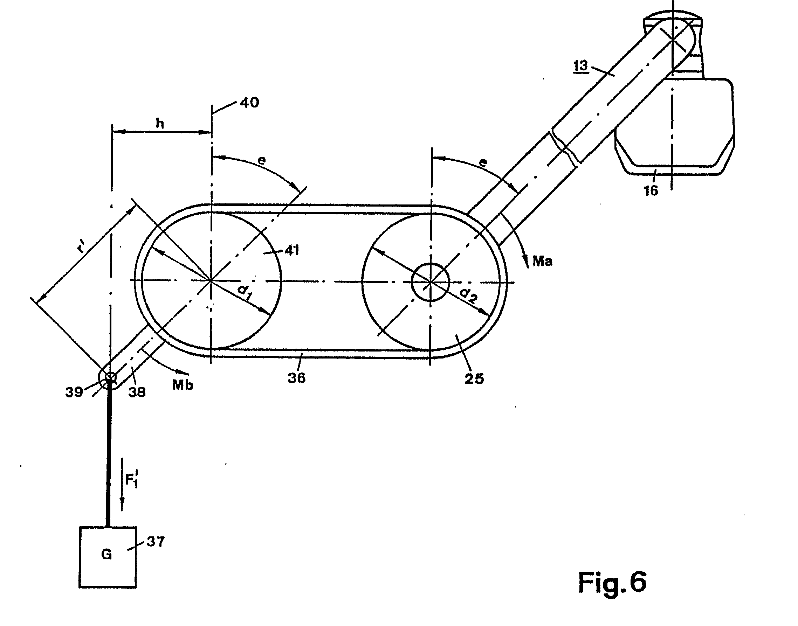

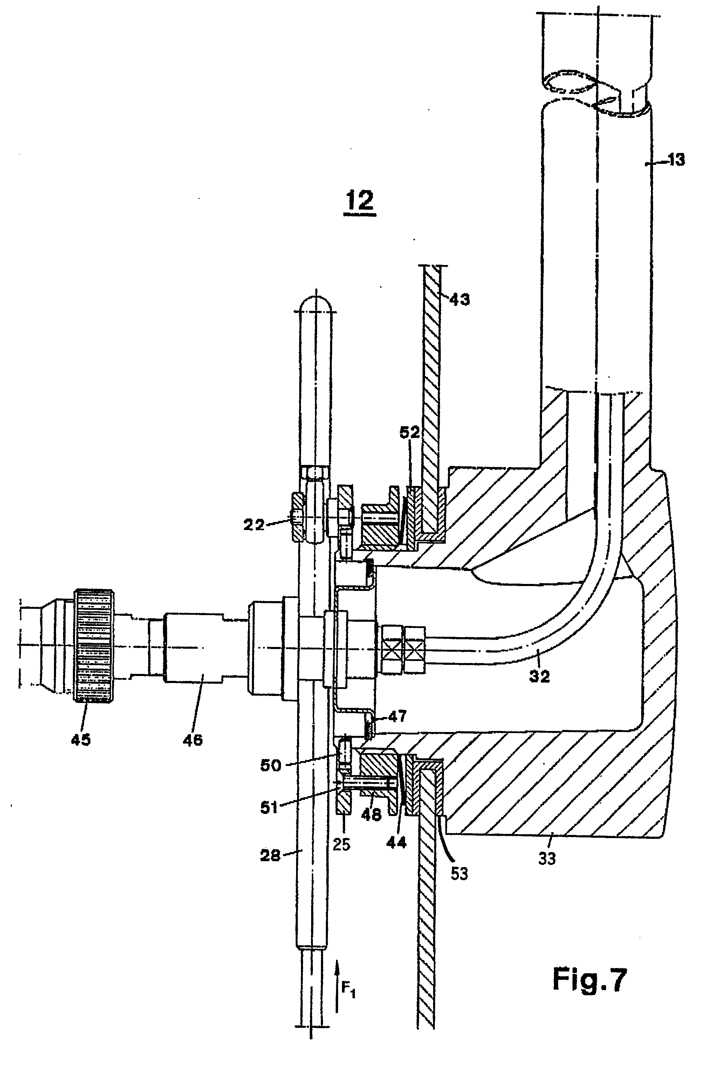

Wie weiter unten anhand Fig. 7 detaillierter beschrieben, wird bei der Haltevorrichtung die Hochfrequenzleitung 32 durch ein zentrales Loch 27 innerhalb des Drehlagers 12 geführt. Die Übertragung der Kraft $F_1$ der Druckluftfeder 23 auf den Drehpunkt 22 darf daher nicht über eine gerade Stange, sondern muß über eine geeignete gekröpfte Stange 28 erfolgen, so daß diese Stange nicht die Hochfrequenzleitung 32 berührt. Statt einer gekröpften Stange 28 kann zur Übertragung der Kraft $F_1$ der Druckluftfeder 23 auch ein schlupffreier Riementrieb, wie in Fig. 6 dargestellt, verwendet werden, wodurch die zentrale Einführung der Hochfrequenzleitung 32 ebenfalls nicht behindert wird.

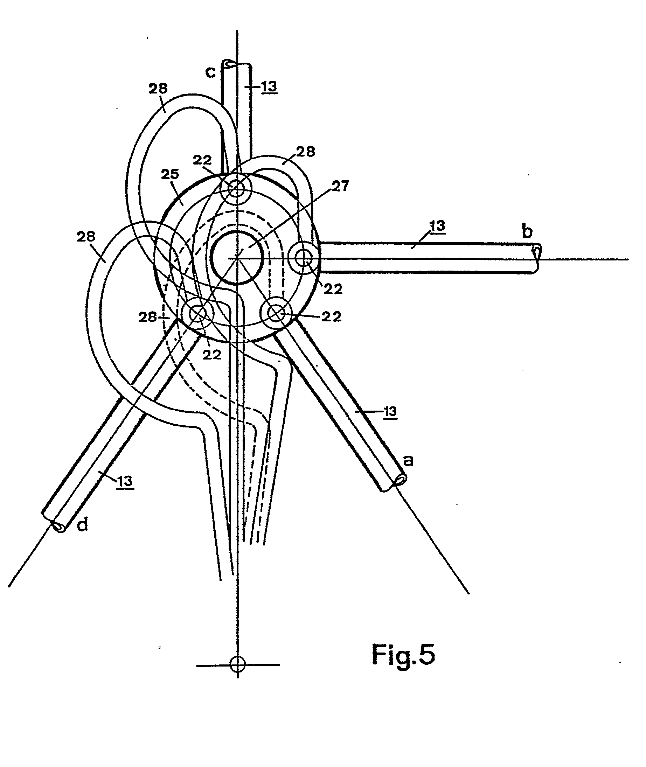

In Fig. 5 ist die Funktion der gekröpften Stange 28 in verschiedenen Winkelstellungen a bis b des Haltearmes 13 dargestellt, so daß der Grund der Kröpfung deutlich erkennbar ist. Die Stange 28 darf das zentrale Loch 27, durch welches koaxial die Hochfrequenzleitung geführt wird, nicht schneiden.

In Fig. 6 ist ein weiteres Ausführungsbeispiel der innerhalb des Gehäuses 1 des Hochfrequenz-Therapiegerätes angeordneten Bestandteile der erfindungsgemäßen Haltevorrichtung dargestellt. Hier wurde das Gegendrehmoment $M_b$ durch ein Gewicht 37, welches den Hebelarm 38 wirkt, erzeugt. Das Drehmoment $M_b$ ergibt sich hierbei aus der Kraft $F_1$ und dem horizontalen Abstand h zwischen dem Angriffspunkt 39 der Kraft $F_1$ und der vertikalen Mittelachse 40 der Riemenscheibe 41. Das Drehmoment $M_b$ wird über einen schlupffreien Riemen, beispielsweise einem Zahnriemen, auf die Scheibe 25 übertragen, wobei das Übersetzungsverhältnis $d_1/d_2 = 1$ sein muß. Auf diese Weise ist gewährleistet, daß der Haltearm 13 bei jedem Winkel 1 ausreichend gut im Gleichgewicht ist.

Allgemein gilt bei beiden Ausführungsbeispielen der Figuren 4 und 6, daß ein Gegendrehmoment $M_b$ innerhalb des Gehäuses des Hochfrequenz-Therapiegerätes vorhanden ist, welches möglichst genau den gleichen Betrag hat wie das Drehmoment $M_a$ des Haltearmes 13 inklusive des jeweils verwendeten Applikators 16, jedoch mit umgekehrtem Vorzeichen bzw. umgekehrter Drehrichtung.

In Fig. 7 ist das Drehgelenk 12 detaillierter dargestellt. Folgende erfindungsrelevante Details des Drehgelenkes 12 sind in Fig. 7 dargestellt. Der Drehgelenkkopf 33, der innen hohl ist, so daß die Hochfrequenzleitung 32 durch ihn hindurchgeführt werden kann, wie übrigens durch den gesamten Haltearm 13 bis hin zu den Applikatoren 16. Die Lagerung des Drehgelenkes 12 in einer Seitenwand 43 des Hochfrequenz-Therapiegerätes 1 besteht aus dem Gleitlager 53, in welchem der Drehgelenkkopf 33 angeordnet ist sowie aus einer Reibungsbremse, bestehend aus der Reibscheibe 52 und der Gegenreibfläche des Gleitlagers 53. Die Reibkraft wird durch eine Tellerfeder 44 erzeugt, welche auf die Reibscheibe 52 drückt und mittels des Justierringes 48 justierbar ist. Die Überleitung der Kraft $F_1$ aus der gekröpften Stange 28 in den Drehgelenkkopf 33 erfolgt über den Drehpunkt 22, die Scheibe 25 und die Stifte 50. Der Justierring 48 wird nach der Justierrung der Kraft der Tellerfeder 44 mittels der Schraube 51 gegen Dejustierung gesichert. Die Hochfrequenzleitung 32 wird innerhalb des Drehgelenkes 12 koaxial durch eine Formscheibe 47 zentriert. Zwischen dem Hochfrequenzgeneratorausgang 45 und der Hochfrequenzleitung 32 ist eine koaxiale Drehkupplung 46 der Hochfrequenzleitung angeordnet.

In Figur 8 ist das axiale Drehgelenk 20 im Bereich $L_2$ des Haltearmes 13 dargestellt. Die koaxiale Hochfrequenzleitung 32 endet imBereich $L_2$ des Haltearmes 13 in einer koaxialen Drehkupplung, bestehend aus dem koaxialen Mittelleiter 59, dem koaxialen Außenleiter 60, der innerhalb des Kupplungsgewindestückes 63 angeordnet ist, welches wiederum in das Übergangsstück 67 zwischen dem Rohr des Haltearmes 13 und der koaxialen Drehkupplung eingeschraubt ist. Das Endstück 57 des Haltearmes 13 ist gegen das Übergangsstück 67 konzentrisch verdrehbar, wobei die Stirnflächen des Endstückes 57 und des Übergangsstückes 67 gegeneinander reiben, wodurch die Drehbewegung des Endstückes gebremst wird. Die Bremswirkung ist vom Reibungskoeffizienten der beiden gegeneinander reibenden Stirnflächen und der Kraft, mit welcher diese beiden Stirnflächen gegeneinander gedrückt werden, abhängig. Diese Kraft wird durch die Tellerfedern 58 erzeugt, deren Spannung durch mehr oder weniger tiefes Einschrauben des Kupplungsgewindestückes 63 in das Übergangsstück 67 justierbar ist. Dieses Drehgelenk 20 ist erforderlich, um die Applikatoren 16, wie in Figur 3 dargestellt, um 360 Grad am Haltearm 13 drehen zu können, wobei die Bremswirkung verhindert, daß die Applikatoren unbeabsichtigt durch ihr eigenes Gewicht oder durch leichtes Berühren durch den Patienten in eine ungeeignete Lage gleiten. Die koaxialen Stützscheiben 61 und 62 zentrieren den Mittelleiter 59 in die Mitte der Drehkupplung. Mittels des Schraubdeckels 64, der am Außendurchmesser Gewinde trägt, werden die koaxialen Stützscheiben 62 und 61 sowie derMittelleiter 59 innerhalb des Kupplungsgewindestückes 63 fest verschraubt. Der Außenleiter der Koaxialleitung 32 wird durch die äußere 65 und innere 66 Klemmhülse elektrisch leitfähig mit dem Außenleiter 60 verbunden, wobei die Klemmung mittels der Klemmschrauben 68 und 69 erfolgt.

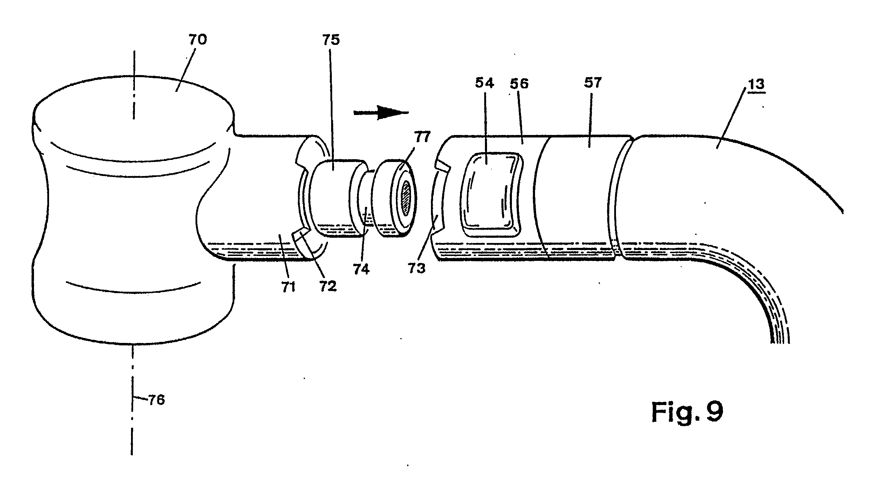

Der in Figur 9 dargestellte Koaxialstecker 75 ist mit einer Nut 74 ausgestattet, in die mittels der Kraft der Druckfeder 55 die Taste 54 einrastet wenn dieser Koaxialstecker 75 so tief in die Koaxialkupplung eingesteckt ist, daß die Nase 72 des Überganges 71 vom Applikatorkopf 70 in die Nut 73 einrastet. Die Nase 72 verhindert, wenn sie in die Nut 73 eingerastet ist, daß der Applikator 16 sich in der koaxialen Drehkupplung 20 unbeabsichtigt verdrehen kann.

Beim Einführen des Koaxialsteckers 75 in die koaxiale Drehkupplung wird die Taste 54 durch die schräge , konzentrische Fläche 77 am Ende des Koaxialsteckers 75 automatisch gegen die Kraft der Druckfeder 55 heruntergedrückt, so daß die Taste 54 in die Nut 74 einrastet. Zum Herausziehen des Koaxialsteckers 75 aus der koaxialen Drehkupplung 20 muß die Taste 54 gegen die Kraft der Druckfeder 55 eingedrückt werden. Die Steckerführung 56 ist fest in das Endstück 57 eingeschraubt. Die Applikatoren sind außendem um die Mittelachse 76 des Applikatorkopfes 70 um 360 Grad drehbar gelagert.

**Patentansprüche**

1. Haltevorrichtung für einen Applikator an einem elektromedizinischen Therapiegerät, mit
1.) einem starren Haltearm (13), der
1.1) L-förmig abgewinkelt ist,
1.2) einen hohlen Innenraum aufweist, durch den
1.3) eine Hochfrequenzleitung (32) verläuft,
1.4) an seinem einen Ende den an die Hochfrequenzleitung anschließbaren Applikator (16) trägt und
1.5) an seinem anderen Ende um eine horizontale Achse (11) um mehr als 180° verschwenkbar an einer Seitenwand des Gerätes (1) mittels eines Drehgelenks (12) gelagert ist, wobei
2.) die Hochfrequenzleitung (32) durch das Drehgelenk (12) verläuft, sowie mit
3.) einer in dem Gerät vorgesehenen Ausgleichseinrichtung (24;23,28;36,37,41), die dem durch den Haltearm (13) in unterschiedlichen Drehlagen (Fig. 3) verursachten unterschiedlich großen Drehmoment ($M_a$) entgegenwirkt.

2. Haltevorrichtung nach Anspruch 1, **dadurch gekennzeichnet**, daß die Ausgleichseinrichtung (23,28;36,37,41) eine das Drehmoment ($M_a$) - entsprechend dem Gewicht des Haltearms (13) und des Applikators (16) multipliziert mit der horizontalen Entfernung des gemeinsamen Schwerpunkts von der horizontalen Drehachse (11) - zumindest angenähert kompensierende Einrichtung ist, die ein einer ebenfalls einer Cosinusfunktion folgendes Gegendrehmoment ($M_b$) erzeugt.

3. Haltevorrichtung nach Anspruch 1 oder 2, **dadurch gekennzeichnet**, daß die Ausgleichseinrichtung (23,28) eine Druckluftfeder (23) oder dergleichen Kraftquelle zur Erzeugung einer mindestens angenähert konstanten Kraft ($F_1$) aufweist, die über eine gekröpfte Stange (28) zur Ausübung des Gegendrehmoments ($M_b$) angreift.

4. Haltevorrichtung nach Anspruch 1 oder 2, **dadurch gekennzeichnet**, daß die Ausgleichseinrichtung (36,37,41) ein Gewicht (37) aufweist, das zur Erzeugung des Gegendrehmoments ($M_b$) über einen Hebelarm (38) an einer Riemenscheibe (41) angreift, und daß das Gegendrehmoment ($M_b$) über einen schlupffreien Riemenantrieb auf eine Scheibe (25) übertragen wird, an der der Haltearm (13) befestigt ist.

5. Haltevorrichtung nach einem der vorhergehenden Ansprüche, **dadurch gekennzeichnet**, daß eine Reibungsbremse (24) vorgesehen ist, die an der Scheibe (25) angreift, an der der Haltearm (13) befestigt ist.

6. Haltevorrichtung nach Anspruch 5, **dadurch gekennzeichnet**, daß die durch die Reibungsbremse (24) ausgeübte Reibkraft einstellbar und aufhebbar ist.

7. Haltevorrichtung nach Anspruch 5 oder 6, **dadurch gekennzeichnet**, daß die Reibungsbremse als Ausgleichseinrichtung vorgesehen ist.

8. Haltevorrichtung nach einem der vorhergehenden Ansprüche, **dadurch gekennzeichnet**, daß der Applikator (16) um mindestens eine Achse um 360° drehbar ist, und daß das Drehgelenk (20) durch Reibkraft in der eingestellten Drehlage gesichert ist.

9. Haltevorrichtung nach einem der vorhergehenden Ansprüche, **dadurch gekennzeichnet**, daß für die Hochfrequenzleitung (32) im Bereich des Drehlagers (12) und des Drehgelenks (20) jeweils eine koaxiale Drehkupplung (46) vorgesehen ist.

10. Haltevorrichtung nach einem der vorhergehenden Ansprüche, **dadurch gekennzeichnet**, daß das Drehlager (12) im Bereich einer oberen Ecke (7) an der breiteren Seitenwand (6) eines quaderförmigen Geräts (1) angeordnet ist.

## Claims

1. Holding device for an applicator on an electro-medical therapy instrument with
   1) a rigid holding arm (13), which
      1.1) is bent in L-shaped manner,
      1.2) has a hollow inner area, through which
      1.3) passes a high frequency line (32),
      1.4) carries at its one end the applicator (16) connectable to the high frequency line and
      1.5) at its other end is mounted so as to be pivotable about its horizontal axis (11) by more than 180° on a side wall of the instrument (1) by means of a pivot joint (12), in which
   2) the high frequency line (32) passes through the pivot joint (12), as well as with
   3) a compensating device (24, 23, 28, 36, 37, 41) provided in the instrument, which counteracts the torque ($M_a$) of varying magnitude caused in different rotation positions (fig. 3) by the holding arm (13).

2. Holding device according to claim 1, characterized in that the compensating device (23, 28, 36, 37, 41) is a device at least approximately compensating the torque ($M_a$), corresponding to the weight of the holding arm (13) and the applicator (16) multiplied by the horizontal distance of the common centre of gravity from the horizontal rotation axis (11) and produces an opposing torque ($M_b$) which also follows a cosine function.

3. Holding device according to claims 1 or 2, characterized in that the compensating device (23, 28) has a compressed air spring (23) or similar force source for producing an at least approximately constant force ($F_1$), which acts by means of a cranked rod (28) for exerting the opposing torque ($M_b$).

4. Holding device according to claims 1 or 2, characterized in that the compensating device (36, 37, 41) has a weight (37), which for producing the opposing torque ($M_b$) acts by means of a lever arm (38) on a belt pulley (41) and that the opposing torque ($M_b$) is transferred by means of a non-slip belt drive to a disk (25), to which is fixed the holding arm (13).

5. Holding device according to one of the preceding claims, characterized in that a friction brake (24) is provided, which acts on the disk (25), to which is fixed the holding arm (13).

6. Holding device according to claim 5, characterized in that the frictional force exerted by the friction brake (24) is adjustable and removable.

7. Holding device according to claims 5 or 6, characterized in that the friction brake is provided as a compensating device.

8. Holding device according to one of the preceding claims, characterized in that the applicator (16) is rotatable by 360° about at least one axis and that the pivot joint (20) is secured in the set rotation position by frictional force.

9. Holding device according to one of the preceding claims, characterized in that in each case one coaxial rotary coupling (46) is provided for the high frequency line (32) in the vicinity of the pivot bearing (12) and the rotary joint (20).

10. Holding device according to one of the preceding claims, characterized in that the pivot bearing (12) is arranged in the vicinity of its upper corner (7) on the wider side wall (6) of a parallelepipedic instrument (1).

## Revendications

1. Dispositif-support d'un applicateur contre un appareil électrothérapie médiacale, comprenant
   1.) un bras-support rigide (13)
      1.1) coudé en L,
      1.2) présentant un volume intérieur creux traversé par
      1.3) une ligne haute fréquence (32),
      1.4) supportant par une de ses extrémités l'applicateur (16) raccordable à la ligne haute fréquence et
      1.5) reposant par l'autre de ses extrémités autor d'un axe horizontal (11) pivotant de plus de 180° contre la paroi latérale de l'appareil (1) au moyen d'une articulation tournant (12) et où
   2.) la ligne haute fréquence (32) traverse cette articulation (12) ainsi que
   3. d'un dispositif d'équilibrage (24; 23, 28; 36, 37, 41) prévue dans l'appareil compensant le couple ($M_a$) important et variable exercé par le bras-support (13) lorsqu'il tourne dans diverses positions (Fig. 3).

2. Dispositif-support selon revendication 1, caractérisé en ce que le dispositif d'équilibrage (23, 28; 36, 37, 41) est un dispositif compensant au moins de façon approximative le couple ($M_a$) – suivant le poids du bras-support (13) et de l'applicateur (16) multiplié par la distance horizontale entre le centre de gravité commun et l'axe horizontal de rotation (11) – en exerçant un couple résistant ($M_b$) obéissant lui aussi à une fonction cosinus.

3. Dispositif-support selon revendication 1 ou 2, caractérisé en ce que le dispositif d'équilibrage (23, 28) présente un ressort pneumatique (23) ou une source d'énergie assimilée servant à engendrer une force (F1) au moins approximativement constante, lequel ressort ou laquelle source exerce le couple résistant via une tringle ondulée (28).

4. Dispositif-support selon revendication 1 ou 2, caractérisé en ce que le dispositif d'équilibrage (36, 37, 41) présente un poids (37) opérant via un bras de levier (38) et une poulie (41) pour développer le couple résistant ($M_b$), et en ce que le couple résistant ($M_b$) est transmis à un disque (25) via un mécanisme à courroie sans glissement, poulie contre laquelle est fixé le bras-support (13).

5. Dispositif-support selon l'une des revendications précédentes, caractérisé en ce qu'un frein à friction (24) est prévu, agissant contre un disque (25) contre lequel est fixé le bras-support (13).

6. Dispositif-support selon revendication 5, caractérisé en ce que l'on peut régler et supprimer la force de friction exercée par le frein à friction.

7. Dispositif-support selon revendication 5 ou 6, caractérisé en ce que le frein à friction est prévu à titre de dispositif d'équilibrage.

8. Dispositif-support selon l'une des revendications précédentes, caractérisé en ce que l'applicateur (16) peut tourner de 360° autour d'au moins un axe, et en ce que l'articulation tournante (20) est retenue par la force de friction dans la position réglée.

9. Dispositif-support selon l'une des revendications précédentes, caractérisé en ce qu'un joint coaxial tournant (46) est prévu pour la ligne haute fréquence (32) dans la zone du coussinet tournant (12) et de l'articulation tournante (20).

10. Dispositif-support selon l'une des revendications précédentes, caractérisé en ce que le coussinet tournant (12) est situé dans la zone correspondant au coin supérieur (7) sur la paroi de plus grande largeur (6) d'un appareil parallélépipédique (1).

Fig.1

Fig.3

Fig.2

Fig.4

EP 0 229 193 B1

Fig.5

Fig. 6

EP 0 229 193 B1

Fig.7

Fig. 8

Fig. 9

EP 0 229 193 B1